# EUROPÄISCHE PATENTSCHRIFT

(11) **EP 1 463 466 B1**
(45) Veröffentlichungstag und Bekanntmachung des Hinweises auf die Patenterteilung: **16.12.2009**
(21) Anmeldenummer: 03702385.0
(22) Anmeldetag: 03.01.2003
(51) Int. Cl.: A61F 2/46

(54) **HILFSMITTEL ZUR IMPLANTATION EINER HÜFTGELENKENDOPROTHESE**
ACCESSORY FOR IMPLANTING A HIP ENDOPROSTHESIS
ACCESSOIRE POUR L'IMPLANTATION D'UNE ENDOPROTHESE DE LA HANCHE

(30) Priorität: 10.01.2002 DE 10200690
(43) Veröffentlichungstag der Anmeldung: 06.10.2004
(73) Patentinhaber: Smith & Nephew Orthopaedics AG, 6343 Rotkreuz (CH)
(72) Erfinder: GRAF, Reinhard, A-8852 Murau (AT); IMHOF, Martin, CH-6343 Rotkreuz (CH); BRACK, René, CH-6330 Cham (CH)
(74) Vertreter: Popp, Eugen
(86) Internationale Anmeldenummer: PCT/EP2003/000035
(87) Internationale Veröffentlichungsnummer: WO 2003/057087

(56) Entgegenhaltungen:
- DE-U- 20 021 494
- FR-A- 2 233 972
- US-A- 6 102 915
- US-A1- 2001 012 967

## Beschreibung

Die Erfindung betrifft ein Hilfsmittel zur Implantation einer Hüftgelenkendoprothese gemäß dem Oberbegriff des Anspruches 1.

Ein solches Hilfsmittel ist aus der US-A-6 102 915 bekannt, wobei dort die Manipulierpfanne gleichzeitig das Fräswerkzeug ist und die Einrichtung zum Festhalten der ausgerichteten Lage der Manipulierpfanne durch Nägel gebildet wird. Wenn jedoch ein System mit von der Manipulierpfanne getrenntem Fräswerkzeug bevorzugt wird, muß der Operateur beim Einsetzen von Hüftendoprothesen verschiedene Arbeitsgänge unter Einsatz von Werkzeugen ausführen, insbesondere auch das natürliche Acetabulum mit dem gesonderten Fräswerkzeug ausfräsen, um eine Lagerschale zu erhalten, in der eine künstliche Hüftpfanne verankert werden kann. Des weiteren kommt ein Pfannen-Einschlaginstrument zum Einsatz. Bei beiden Werkzeugen muß der Operateur auf eine möglichst genaue Ausrichtung der Werkzeuge achten, damit die vorgesehene Positionierung der Hüftpfanne möglichst genau erreicht werden kann.

Wichtige Hilfsmittel, die die richtige Positionierung bzw. Ausrichtung der Werkzeuge unterstützen, sind sog. Navigationssysteme, die Computer-assistiert arbeiten. Es ist augenscheinlich, daß der Aufwand für derartige Systeme erheblich ist. Dementsprechend sind auch die Kosten unter Anwendung derartiger Systeme relativ hoch. Im Hinblick darauf, daß es auch in der Medizin gilt, die Kosten zu reduzieren, ohne daß die Qualität der medizinischen Versorgung darunter leidet, liegt der vorliegenden Erfindung die Aufgabe zugrunde, ein Hilfsmittel zur Implantation einer Hüftgelenkendoprothese zur Verfügung zu stellen, welches mit einfachen mechanischen Mitteln eine exakte Positionierung der Hüftpfanne in Relation zum Femur bzw. zu dem im Femur verankerten Gelenkkopf erlaubt. Dabei muß darauf geachtet werden, daß die Pfanne so implantiert wird, daß bei jeder nur denkbaren Bewegung des Femur eine Kollision zwischen Pfannenrand und Schenkelhals vermieden wird.

Diese Aufgabe wird erfindungsgemäß durch ein Hilfsmittel der eingangs genannten Art gelöst, welches gemäß dem kennzeichnenden Teil des Anspruches 1 weitergebildet ist.

Der Kern des erfindungsgemäßen Hilfsmittels liegt also darin, daß mittels eines Manipulier-Gelenkkopfes die Manipulierpfanne in solche Lage innerhalb des Acetabulums gebracht wird, daß bei allen denkbaren Bewegungen des Femur eine Kollision des Pfannenrandes mit dem Schenkelhals ausgeschlossen ist. Zu diesem Zweck ist der Manipulier-Gelenkkopf mit entsprechenden mechanischen oder optischen Ausrichtmitteln versehen. Bei einem ersten bevorzugten Ausführungsbeispiel sind die Ausrichtmittel in Form einer über den sphärischen Teil des Manipulier-Gelenkkopfes radial nach außen vorstehenden Schulter ausgebildet. Diese Schulter korrespondiert zur Ausrichtung der Manipulierpfanne im Acetabulum mit dem Öffnungsrand derselben. In "Nullstellung" des Femur ist die Schulter am Manipulier-Gelenkkopf vom Öffnungsrand der Manipulierpfanne über den Umfang desselben etwa gleichmäßig beabstandet. Der Manipulier-Gelenkkopf ist zum Zwecke der Ausrichtung der Manipulierpfanne am Hals einer Manipulierraspel befestigt, insbesondere auf diesen aufgesteckt. Die Manipulierraspel selbst ist innerhalb des Femur fixiert. Anschließend führt der Operateur sämtliche denkbaren Bewegungen des Femur durch wie folgt:
- Flexion/Extension um die sog. "medio-laterale" Achse
- Abduktion/Adduktion um die sog. "anterior/posteriore" Achse
- Innen-/Außenrotation um die sog. "cranio-caudale" Achse

Durch die dabei auftretende Kollision zwischen Schulter des Manipulier-Gelenkkopfes und Öffnungsrand der Manipulierpfanne wird die Manipulierpfanne in eine Position gebracht, in der nach der endgültigen Implantation der Hüftgelenkendoprothese eine Kollision zwischen Öffnungsrand der Hüftpfanne und dem Schenkelhals sicher vermieden wird.

Natürlich bedarf es dann auch noch einer Einrichtung zum Festhalten der ausgerichteten Lage der Manipulierpfanne, wobei mittels dieser Einrichtung dann ein Knochenfräser und ein Einschlaginstrument für die Plazierung der Hüftpfanne innerhalb der im Acetabulum ausgefrästen Lagerschale entsprechend ausrichtbar sind.

Die erwähnte Schulter am Manipulier-Gelenkkopf kann auch durch etwa gleichmäßig über dem Umfang verteilt angeordnete Schulterabschnitte definiert sein. Diese Abschnitte könnten im Extremfall auch durch stiftartige Vorsprünge ersetzt werden. Natürlich bedarf es dann einer ausreichenden Anzahl von Vorsprüngen, um das oben beschriebene Ausrichten der Manipulierpfanne zu erreichen.

Als Einrichtung zum Festhalten der ausgerichteten Lage der Manipulierpfanne dient vorzugsweise ein im Knochen fixierbarer Führungsstab, der mit einer an der Manipulierpfanne angeordneten Führungseinrichtung korrespondiert. Der Führungsstab kann entweder als Nagel oder auch als Gewindestab ausgebildet sein. Im letztgenannten Fall weist der Führungsstab an dem im Knochen verankerbaren Endabschnitt ein Schraubgewinde auf, so daß er in dem Knochen, nämlich Beckenknochen einschraubbar ist.

Die dem Führungsstab zugeordnete Führungseinrichtung an der Manipulierpfanne umfaßt vorzugsweise ein über einen Arm mit der Manipulierpfanne verbundenes Bauteil, insbesondere einen Führungsblock oder eine Führungshülse mit einer Bohrung zur Aufnahme und Führung des Führungsstabes. Dementsprechend wird nach Ausrichtung der Manipulierpfanne der Führungsstab durch die Führungsbohrung in der an der Manipulierpfanne angeordneten Führungseinrichtung hindurchgeführt und im Knochen verankert. Anschließend wird die Manipulierpfanne vom Führungsstab entfernt. Der Führungsstab ist dann frei zur Befestigung, insbesondere zum Aufschieben einer Lehre zur Ausrichtung eines Fräskopfes bzw. dessen Antriebsachse so, daß die Ausrichtung des Fräskopfes derjenigen der Manipulierpfanne entspricht. Bei Anordnung nur eines einzigen Führungsstabes ist die Ausrichtlehre vorzugsweise um diesen auch noch verschwenkbar.

Bei einer bevorzugten Ausführungsform des ersten Ausführungsbeispiels umfaßt die Ausrichtlehre einen auf den Führungsstab aufschiebbaren Arm, insbesondere Bügel, an dessen freiem, d.h. dem Führungsstab entgegengesetzten Ende eine Richtplatte, insbesondere mit Markierungen versehene Richtplatte zur Ausrichtung der Fräser-Antriebsachse angeordnet ist, wobei zur Ausrichtung der Fräser-Antriebsachse diese in volle bzw. spaltfreie Anlage an die Richtplatte und ggf. noch parallel zu dieser verschwenkt wird. Die auf der Richtplatte vorzugsweise noch vorgesehenen Markierungen erlauben ein Verschwenken der Fräser-Antriebsachse parallel zur Richtplatte in eine Lage entsprechend einer vorgegebenen Markierung, insbesondere vorgegebenen Nullposition. Dieser Nullposition können zwei maximale Toleranz-Positionen von ± 5° zugeordnet sein.

Um die Richtplatte auch während der Betätigung des Fräsers spaltfrei an der Antriebsachse desselben halten zu können, ist die Fräser-Antriebsachse mit einer Drehhülse versehen, innerhalb der die Fräser-Antriebsachse drehbar gelagert und an der die Richtplatte auch während des Fräsens in spaltfreier Anlage gehalten werden kann.

Wie bereits oben erwähnt, ist auch noch ein Pfanneneinschlaginstrument zur endgültigen Plazierung der Hüftpfanne in einer vorgegebenen Ausrichtung vorgesehen. Das Pfanneneinschlaginstrument ist ebenfalls an der erwähnten Richtplatte der Ausrichtlehre ausrichtbar, und zwar in gleicher Weise wie der Fräskopf bzw. dessen Antriebsachse. Da es sich bei dem Pfanneneinschlaginstrument per se um ein an sich bekanntes Instrument handelt, bedarf es hier keiner weiteren Beschreibung.

Die Manipulierpfanne kann auch mit einer Führungseinrichtung für zwei oder mehr parallel zueinander in Knochen fixierbare Führungsstäbe versehen sein. Die Ausrichtlehre für den Fräskopf bzw. dessen Antriebsachse sowie das Pfannen-Einschlaginstrument weist dann ebenfalls zwei oder drei entsprechende Durchgangsbohrungen zum Aufschieben auf die im Knochen fixierten Führungsstäbe auf.

Eine weitere Ausführungsform des ersten Ausführungsbeispiels ist dadurch gekennzeichnet, daß die Richtplatte der Ausrichtlehre U-förmig gebogen ist, wobei der Raum zwischen den beiden Plattenschenkeln zur Aufnahme der Fräser-Antriebsachse dient, und zwar vorzugsweise zur im wesentlichen spielfreien Aufnahme derselben, so daß der Operateur nur noch darauf achten muß, daß die Fräser-Antriebsachse sich in Nullposition parallel zur Richtplatte befindet. Um diese Nullposition besser erkennen zu können, kann der dem Operateur zugewandte, insbesondere obere Plattenschenkel stirnseitig mit Einkerbungen versehen sein, die als Markierungen für die Ausrichtung der Fräser-Antriebsachse parallel zur Richtplatte dienen.

Ein weiteres Ausführungsbeispiel eines erfindungsgemäßen Hilfsmittels ist dadurch gekennzeichnet, daß als Mittel zur Ausrichtung der Manipulierpfanne im Acetabulum optische Erkennungsmittel dienen, z.B. in Form einer sich über den Umfang des sphärischen Teils des Manipulier-Gelenkkopfes erstreckenden Kerbe oder Nut. Statt einer Kerbe oder Nut kann auch eine Markierungslinie vorgesehen sein. In jedem Fall erstreckt sich die erwähnte Markierung innerhalb einer Ebene, die sich entweder senkrecht oder in einem vorbestimmten Winkel zur Gelenkkopf-Mittenachse erstreckt. Im erstgenannten Fall ist die Manipulierpfanne dann richtig ausgerichtet, wenn die Markierung über den Rand der Manipulierpfanne sichtbar wird. Im zweitgenannten Fall ist die Ausrichtung der Manipulierpfanne anatomisch korrekt, wenn die Markierung allseitig, d.h. über den Umfang der Manipulierpfanne nicht mehr erkennbar ist.

In jedem Fall ist der Manipulier-Gelenkkopf so auf den Hals einer Manipulierraspel befestigt, daß die Gelenkkopf-Mittenachse mit der Halsachse zusammenfällt.

Bei einem dritten Ausführungsbeispiel dient als Mittel zur Ausrichtung der Manipulierpfanne im Acetabulum eine sich in einer Ebene senkrecht zur Gelenkkopf-Mittenachse erstreckende Umfangsschulter in Verbindung mit einer sich schräg zur Gelenkkopf-Mittenachse erstreckenden Aufnahme für den Hals einer Manipulierraspel, dessen Längsachse sich parallel zur Schenkelhalsachse erstreckt.

Die Manipulierpfanne ist bei diesem Ausführungsbeispiel dann korrekt ausgerichtet, wenn die Umfangsschulter des Manipulier-Gelenkkopfes bündig ist mit der äußeren Umfangsringfläche der Manipulierpfanne.

Als Einrichtung zum Festhalten der ausgerichteten Lage der Manipulierpfanne können auch gesonderte Fixierstäbe dienen, die sich durch eine Haltevorrichtung für die Manipulierpfanne hindurcherstrecken. Die Fixierstäbe sind an den im Knochen verankerbaren Endabschnitten mit Schraubgewinden versehen, so daß sie in den Knochen einschraubbar sind. An der vorgenannten Haltevorrichtung ist dann vorzugsweise ein Führungsstab anschließbar, und zwar derart, daß dieser sich etwa parallel zur Manipulierpfannen-Mittenachse erstreckt. An diesem Führungsstab kann dann ein Führungselement angeordnet werden, welches zur Führung einer Fräser-Antriebsachse oder eines Pfannen-Einschlaginstruments dient. Durch das Führungselement ist sichergestellt, daß die Ausrichtung der Fräser-Antriebsachse und des Pfannen-Einschlaginstruments derjenigen der Manipulierpfanne entspricht.

Bezüglich weiterer Details dieser Ausführungsform wird auf die entsprechenden Unteransprüche verwiesen.

Nachstehend werden Ausführungsbeispiele des erfindungsgemäßen Hilfsmittels bzw. Ausrichtinstruments für Manipulierpfannen anhand der beigefügten Zeichnung näher erläutert. Diese zeigen in:
- Figur 1: am Hals einer innerhalb des Femur plazierten Manipulierraspel befestigten Manipulier-Gelenkkopf in Zuordnung zum natürlichen Acetabulum;
- Figur 2: das Ausrichten einer im Acetabulum plazierten Manipulierpfanne mittels des Manipulier-Gelenkkopfes gemäß Figur 1;
- Figur 3: das Plazieren eines Führungsstabes innerhalb einer der Manipulierpfanne zugeordneten Führungseinrichtung zum Festhalten der ausgerichteten Lage der Manipulierpfanne, wobei der Führungsstab zu diesem Zweck im Beckenknochen verankert wird;
- Figur 4 und 5: die Entfernung der Manipulierpfanne aus dem Acetabulum und vom im Knochen verankerten Führungsstab;
- Figur 6: die Plazierung eines Knochenfräsers im Acetabulum sowie das Aufschieben einer Ausrichtlehre für den Knochenfräser bzw. dessen Antriebsachse auf dem im Knochen verankerten Führungsstab;
- Figur 7 und 8: die gegenseitige Ausrichtung von Ausrichtlehre und Fräser-Antriebsachse sowie die Fixierung dieser Relativlage mittels einer Hand des Operateurs oder einer Hilfsperson;
- Figur 9: gegenseitige Ausrichtung von Ausrichtlehre und Pfanneneinschlaginstrument entsprechend der Ausrichtung zwischen Ausrichtlehre und Fräser-Antriebsachse gemäß den Figuren 7 und 8;
- Figur 10: das gesamte Instrumentarium für die Implantation einer Hüftgelenkendoprothese in perspektivischer Ansicht;
- Figur 11: die Manipulierpfanne samt Führungseinrichtung für einen im Knochen verankerbaren Führungsstab in perspektivischer Seitenansicht;
- Figur 12: eine abgewandelte Ausführungsform einer Ausrichtlehre in perspektivischer Ansicht;
- Figur 13: eine weitere Ausführungsform einer Einrichtung zum Festhalten der ausgerichteten Lage der Manipulierpfanne in perspektivischer Ansicht;
- Figur 14: ein zweites Ausführungsbeispiel eines Manipulier-Gelenkkopfes in Zuordnung zu einer Manipulierpfanne im schematischen Schnitt;

- Figur 15: ein drittes Ausführungsbeispiel eines Manipulier-Gelenkkopfes in Zuordnung zu einer Manipulierpfanne im schematischen Schnitt; und
- Figur 16: die Anordnung eines Führungsstabes an einer Manipulierpfannen-Haltevorrichtung sowie die Zuordnung eines Führungselements am erwähnten Führungsstab für die Führung eines Pfannen-Einschlaginstruments in einer Lage entsprechend der ausgerichteten Lage der Manipulierpfanne, in perspektivischer Darstellung.

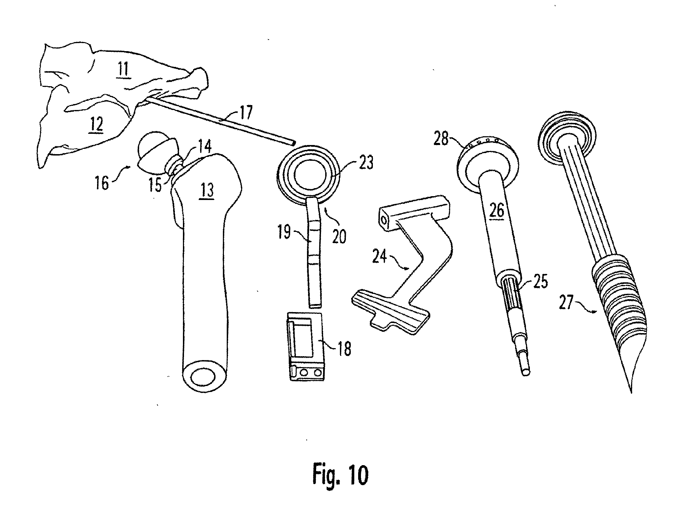

Zunächst sei unter Bezugnahme auf Figur 10 für ein erstes Ausführungsbeispiel das gesamte Instrumentarium für eine anatomiegerechte Implantation einer Hüftgelenkendoprothese dargestellt. Figur 10 zeigt von links nach rechts folgende Teile:

| | |
|---|---|
| 11 | Beckenknochen |
| 12 | Acetabulum |
| 13 | Proximaler Abschnitt eines Femur, innerhalb dem eine nicht näher dargestellte Manipulierraspel fixiert ist |
| 14 | Proximales Ende der innerhalb des Femur plazierten Manipulierraspel |
| 15 | Prothesenhals |
| 16 | Manipulier-Gelenkkopf, der am Prothesenhals in herkömmlicher Weise aufgesteckt ist, insbesondere mittels einer sog. "Steck-Konus-Verbindung" |
| 17 | im Beckenknochen verankerter Führungsstab |
| 18 | an einer Manipulierpfanne befestigbarer Führungsblock für den Führungsstab 17 |
| 20 | Manipulierpfanne mit Haltebügel 19 für den Führungsblock 18 |
| 24 | Ausrichtlehre |
| 25 | Fräser-Antriebsachse |
| 26 | Drehhülse |
| 27 | Pfanneneinschlaginstrument |
| 28 | Fräserkopf |

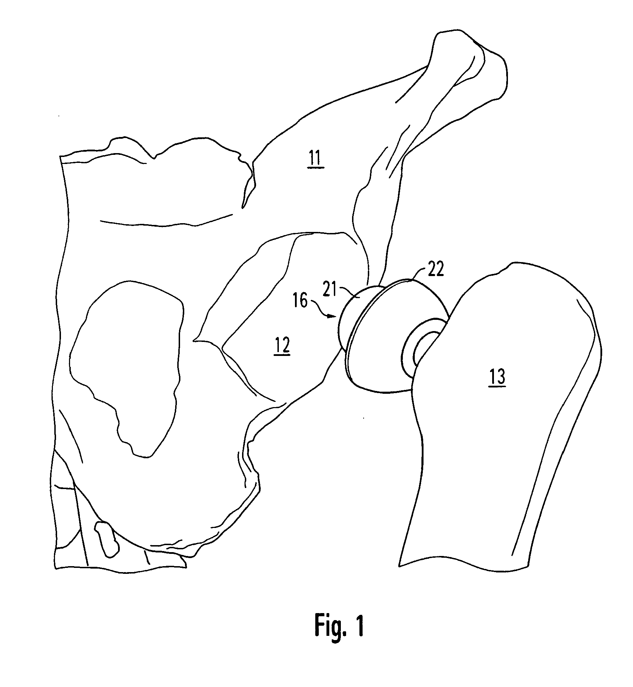

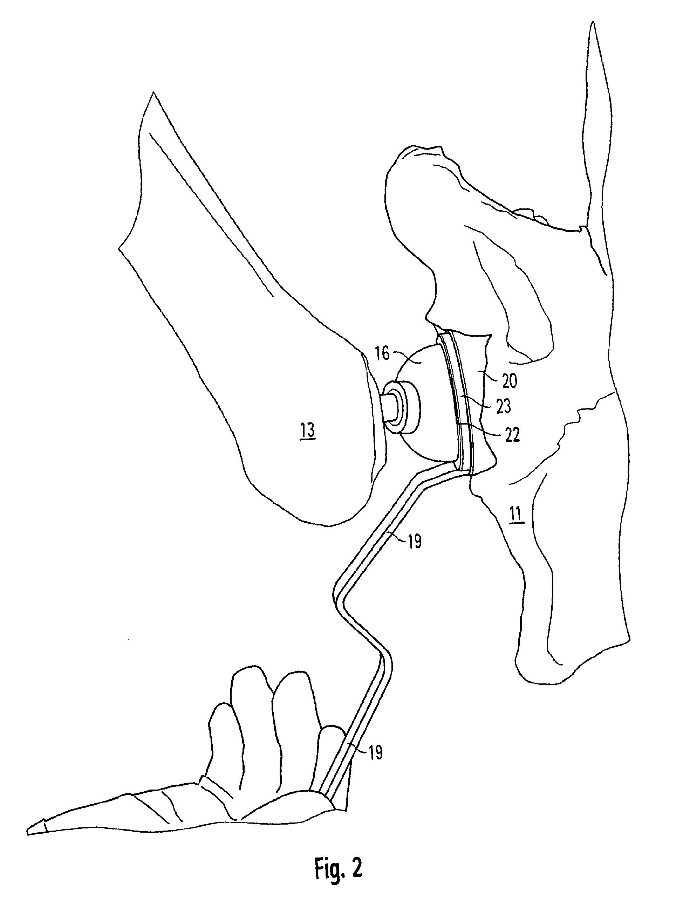

Entsprechend Figur 1 wird zunächst der Schenkelhals reflektiert und ausgehend von der Resektionsebene in den Femur eine Manipulierraspel eingesetzt, an deren Hals ein Manipulier-Gelenkkopf 16 aufgesteckt wird. Der Manipulier-Gelenkkopf 16 umfaßt einen sphärischen Teil 21, über den eine sich über den Umfang erstreckende Schulter 22 radial nach außen vorsteht. Diese Schulter 22 dient zur Korrespondenz mit dem Öffnungsrand 23, der Manipulierpfanne 20 entsprechend Figur 2. In einer "Nullstellung" ist der Abstand der Schulter 22 vom Öffnungsrand 23 über den Umfang des Öffnungsrandes 23 etwa gleich groß. Ausgehend von dieser Nullstellung wird der Femur 13 samt Manipulier-Gelenkkopf 16 nach allen anatomisch denkbaren Richtungen bewegt, so wie eingangs beschrieben. Aufgrund dieser Bewegung kommt es sehr wahrscheinlich an mehreren Stellen zur Kollision zwischen der Schulter 22 und dem Öffnungsrand 23 der Manipulierpfanne 20 mit der Folge, daß die Manipulierpfanne 20 dann innerhalb des Acetabulums entsprechend ausgerichtet wird.

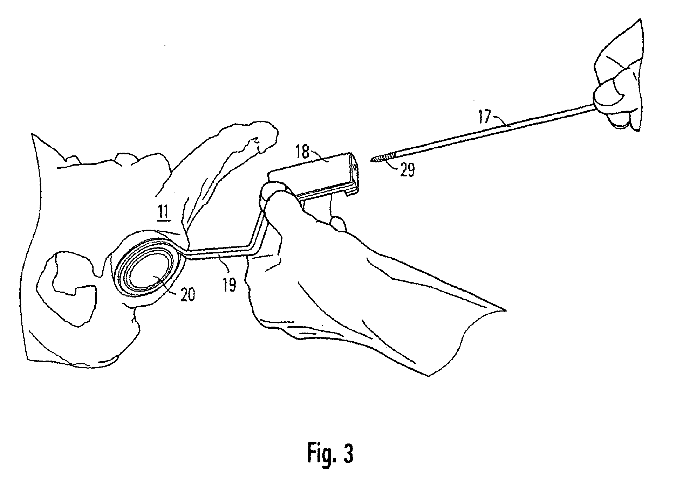

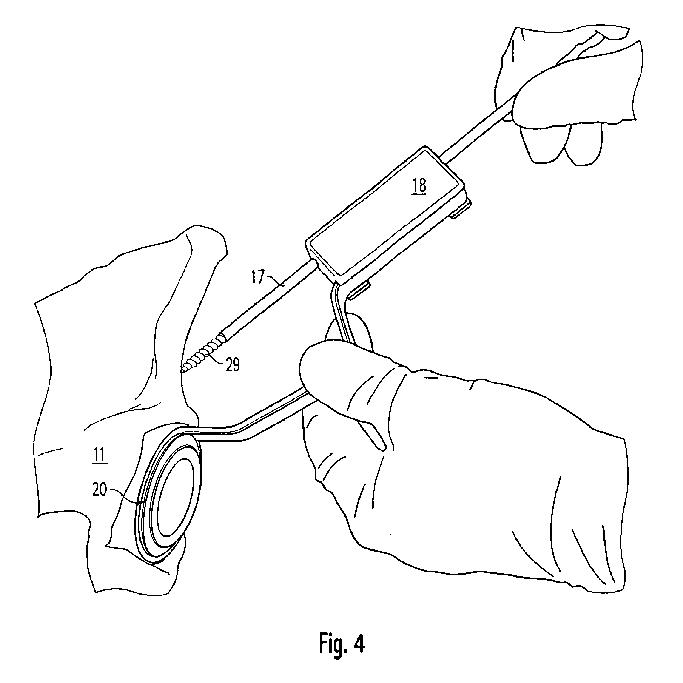

Die ausgerichtete Lage der Manipulierpfanne 20 muß für die entsprechende Ausrichtung sowohl des Knochenfräsers als auch des Pfannen-Einschlaginstruments festgehalten werden. Zu diesem Zweck ist die Manipulierpfanne 20 über einen Haltebügel 19 mit einer Führungseinrichtung in Form eines Führungsblocks 18 verbunden, der eine Führungsbohrung aufweist, dessen Achse außerhalb des Kollisionsbereichs mit der Manipulierpfanne liegt und auf den Beckenknochen 11 gerichtet ist. Durch die erwähnte Führungsbohrung wird ein Führungsstab 17 entsprechend den Figuren 3 und 4 hindurchgeführt. Der Führungsstab 17 weist an seinem dem Knochen 11 zugewandten Endabschnitt ein Gewinde 29 auf, mittels dem der Führungsstab 17 in den Knochen 11 eingeschraubt und darin fixiert werden kann. Der Führungsstab 17 wird natürlich erst nach Ausrichtung der Manipulierpfanne 20 in den Knochen 11 eingeschraubt, so daß damit die Lage der Manipulierpfanne innerhalb des Acetabulums festgehalten werden kann.

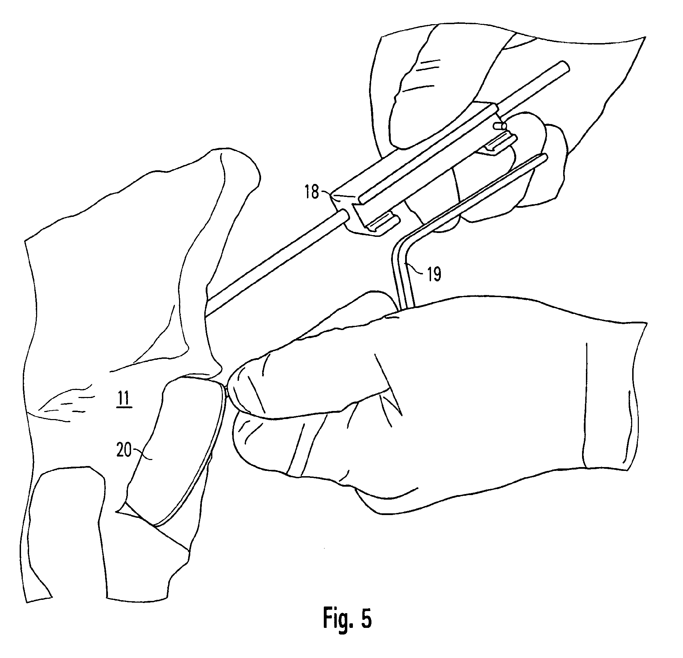

Nachdem der Führungsstab 17 innerhalb des Beckenknochens 11 in der beschriebenen Weise fixiert ist, wird die Manipulierpfanne entsprechend Figur 5 vom Führungsblock 18 abgeklappt und aus dem Acetabulum entfernt.

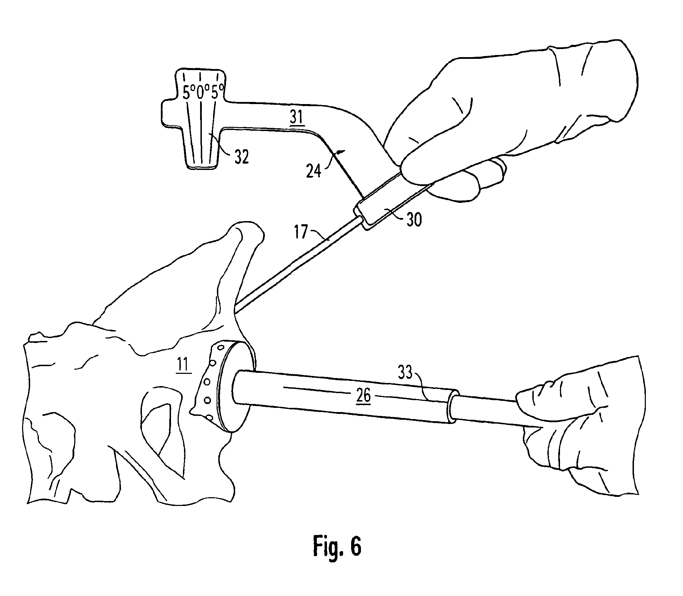

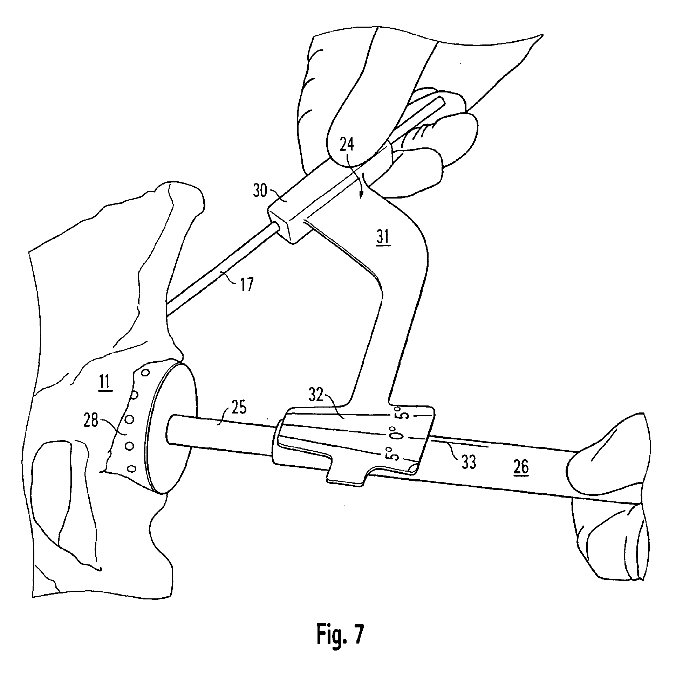

Anschließend wird entsprechend den Figuren 6 und 7 eine Ausrichtlehre 24 auf den Führungsstab 17 aufgeschoben. Die Ausrichtlehre 24 umfaßt dementsprechend eine dem Führungsstab 17 zugeordnete Führungshülse, an der ein Arm, hier Bügel 31 befestigt ist. Am freien Ende des Bügels 31 ist eine Richtplatte 32 ausgebildet. Diese Richtplatte 32 ist mit Markierungen 33 versehen, und zwar mit einer mittleren Null-Markierung und zwei Toleranz-Markierungen ± 5°. Diese Markierungen sind in den Figuren 6 und 7 mit "0°" und "5°" gekennzeichnet.

Diese Ausrichtlehre dient zur Ausrichtung zunächst eines Knochenfräsers mit hemi-sphärischem Fräskopf 28 und Fräser-Antriebsachse 25. Zur Ausrichtung des Fräskopfes bzw. dessen Antriebsachse 25 wird diese in volle bzw. spaltfreie Anlage an die auf die Fräsachse 25 um den Führungsstab 17 verschwenkte Richtplatte 32 und parallel zu dieser in eine Lage entsprechend einer vorgegebenen Markierung, vorzugsweise vorgegebenen Nullposition "0°" verschwenkt, so wie dies Figur 7 gut erkennen läßt.

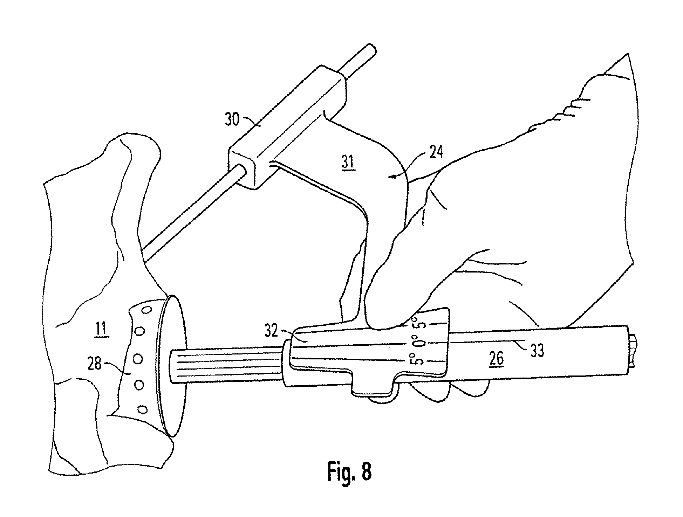

Um eine Kollision zwischen Richtplatte 32 und Fräser-Antriebsachse 25 beim Antrieb des Knochenfräsers zu vermeiden, ist auf die Fräser-Antriebsachse 25 noch eine Hülse 26 aufgesteckt, innerhalb der die Antriebsachse 25 drehbar gelagert und an der die Richtplatte 32 in spaltfreie Anlage bring- und während des Fräsens haltbar ist, so wie dies Figur 8 erkennen läßt.

In der Stellung gemäß Figur 8 kann das Acetabulum in herkömmlicher Weise gefräst werden. Der hemi-sphärische Fräserkopf 28 befindet sich dank Führungsstab und Ausrichtlehre 24 in einer Lage entsprechend der vorher eingestellten anatomiegerechten Lage der Manipulierpfanne 20.

Um die Nullposition der Fräser-Antriebsachse 25 besser einstellen zu können, befindet sich auf der Drehhülse 26 ebenfalls eine sich in Längsrichtung derselben erstreckende Markierung 33. Diese Markierung wird vorzugsweise in Fluchtung mit der Null-Markierung "0°" auf der Richtplatte 32 gebracht. Anschließend kann dann der Fräsvorgang durchgeführt werden, um eine geeignete Lagerschale zum Einsetzen der Hüftpfanne zu erhalten. Die Hüftpfanne wird letztlich in diese Lagerschale entweder eingeschraubt oder mit sog. "Preß-fit" verankert.

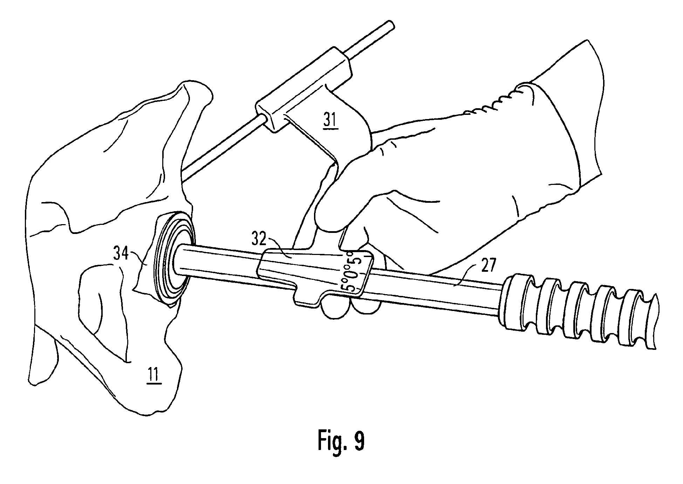

Nach Fräsung der Lagerschale im Acetabulum wird mittels des bereits anhand der Figur 9 und 10 dargestellten Einschlaginstruments 27 die endgültig zu implantierende Hüftpfanne 34 eingeschlagen, wobei natürlich auch dabei darauf geachtet werden muß, daß die Hüftpfanne so eingeschlagen wird, daß sie letztlich eine Lage einnimmt entsprechend der Manipulierpfanne 20. Daher muß auch das Pfannen-Einschlaginstrument 27 ähnlich ausgerichtet werden wie der Knochenfräser bzw. dessen Antriebsachse. Die entsprechende Ausrichtung des Einschlaginstruments 27 ist in Figur 9 dargestellt. Auch hier wird also wieder die Verbindungsachse zwischen Einschlagkopf und Schlagende in spaltfreie Anlage an die Richtplatte 32 gebracht, und zwar vorzugsweise an derselben Markierung wie die Fräser-Antriebsachse. Dann ist gewährleistet, daß die Hüftpfanne 34 anatomiegerecht ausgerichtet in die vorher ausgefräste Lagerschale des Acetabulums eingeschlagen wird.

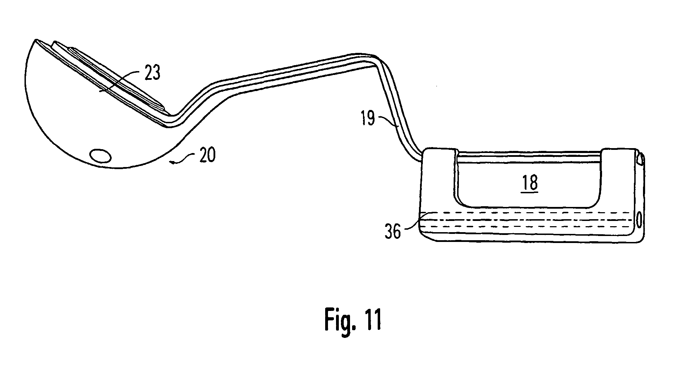

In Figur 11 ist nochmals die Manipulierpfanne mit Führungsblock für den Führungsstab 17 in perspektivischer Seitenansicht dargestellt. Die Führungsbohrung im Führungselement 30 ist gestrichelt dargestellt und mit der Bezugsziffer 36 versehen.

Der Führungselement 30 ist, wie Figur 10, aber auch Figur 5 erkennen lassen, vom Haltebügel 19 abklappbar. Die Verbindung zwischen Haltebügel 19 und Führungsblock ist vorzugsweise als Rastverbindung ausgebildet.

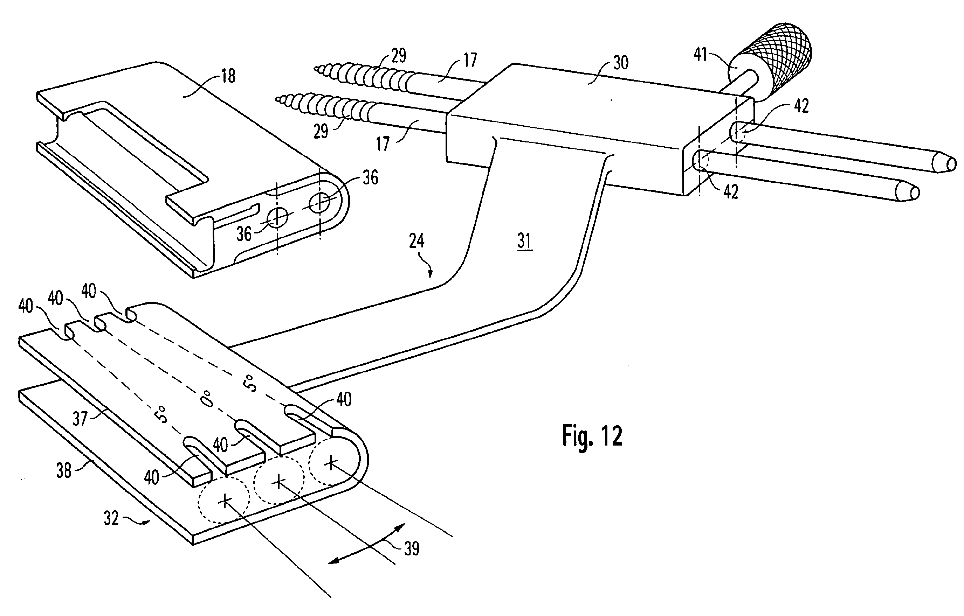

Anhand der Figur 12 wird eine abgewandelte Ausführungsform einer Ausrichtlehre 24 beschrieben, die sich zum einen dadurch auszeichnet, daß das Führungselement 30 zwei Durchgangsbohrungen 42 für die Aufnahme von zwei sich parallel zueinander erstreckenden Führungsstäben 17 aufweist. Des weiteren zeichnet sich die Ausrichtlehre 24 gemäß Figur 12 noch dadurch aus, daß die Richtplatte 32 U-förmig gebogen ist, wobei der Raum zwischen den beiden Plattenschenkeln 37, 38 zur Aufnahme der Fräser-Antriebsachse 25 und/oder des Pfanneneinschlaginstruments 27 dient. Dabei sind diese Instrumentarien zwischen den beiden Plattenschenkeln 37, 38 nur in einer Ebene parallel dazu verschwenkbar entsprechend dem Doppelpfeil 39 in Figur 12. Durch die Anordnung von zwei Führungsstäben ist die Lage der Ausrichtlehre 24 relativ zum Acetabulum eindeutig festgelegt. Es bedarf dann für den Operateur nur noch, die Fräserantriebsachse 25 und/oder das Pfanneneinschlaginstrument 27 zwischen den beiden Plattenschenkeln 37, 38 in einer Ebene parallel dazu zu positionieren. Zur Erleichterung dieser Positionierung sind am oberen Plattenschenkel 37 stirnseitig Einkerbungen 40 vorgesehen. Diese entsprechen den vorgenannten Markierungen "0°" und "± 5°"-Markierungen.

Das Führungselement 30 weist noch eine Feststellschraube 41 zur Fixierung der Ausrichtlehre 24 an den Führungsstäben 17 auf. Aufgrund der Verwendung von zwei Führungsstäben 17 ist es natürlich auch erforderlich, den der Manipulierpfanne 20 zugeordneten Führungsblock 18 mit zwei Durchgangsbohrungen 42 für die Führungsstäbe 17 auszubilden, so wie dies ebenfalls in Figur 12 dargestellt ist.

Die Ausführungsform nach Figur 12 läßt - wie oben ausgeführt - eine Winkelkorrektur der Fräser-Antriebsachse und/oder des Pfannen-Einschlaginstruments nur in einer Ebene zu. Die Führungsstäbe 17 können unterschiedlich lang ausgebildet sein. Sie weisen vorzugsweise ebenfalls wieder ein Gewinde 29 am knochenseitigen Ende auf.

Selbstverständlich werden nach Plazierung der Hüftpfanne 34 die Führungsstäbe 17 aus dem Knochen wieder entfernt. Als Führungsstäbe 17 dienen vorzugsweise sog. "Kirschner-Drähte".

Selbstverständlich muß auch der der Manipulierpfanne zugeordnete Führungsblock 18 der Verwendung von zwei Führungsstäben 17 angepaßt sein, so wie dies ebenfalls in Figur 12 dargestellt ist (Führungsblock 18 mit zwei Durchgangsbohrungen 36 für die Stäbe 17).

Auch wird der Manipulier-Gelenkkopf samt Manipulierraspel aus dem Femur entfernt und durch den endgültigen Hüftschaft samt Gelenkkopf ersetzt. Dann kann das Hüftgelenk in herkömmlicher Weise wieder zusammengesetzt werden. Aufgrund der beschriebenen Manipulation bzw. Ausrichtung ist dann sichergestellt, daß eine Kollision zwischen Prothesenhals und Öffnungsrand der Hüftpfanne 34 ausgeschlossen ist.

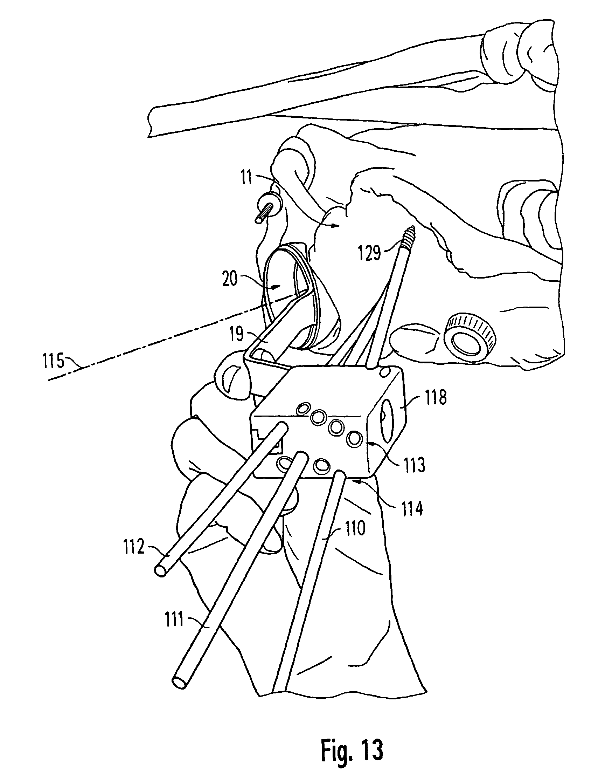

Figur 13 zeigt eine abgewandelte Ausführungsform für eine Einrichtung zum Festhalten der ausgerichteten Lage der Manipulierpfanne 20, wobei diese Einrichtung drei Fixierstäbe 110, 111, 112 aufweist, die sich durch eine Haltevorrichtung 118 hindurch erstrecken, und zwar jeweils im Winkel zueinander. Die Fixierstäbe 110, 111, 112 weisen an den im Knochen verankerbaren Endabschnitten Schraubgewinde 129 auf, so daß sie in den Knochen 11 einschraubbar sind. Die Haltevorrichtung 118 weist im vorliegenden Fall zwei Lochreihen 113, 114 für die Fixierstäbe 110 ff auf, so daß eine ausreichende Auswahl für eine optimale Plazierung der Fixierstäbe 110 ff im Knochen 11 vorhanden ist. An der Haltevorrichtung 118 ist über den Haltebügel 19 die Manipulierpfanne 20 befestigt. Darüber hinaus dient die Haltevorrichtung 118 zum Anschluß eines Führungsstabes 117 derart, daß dieser sich etwa parallel zur Manipulierpfannen-Mittenachse 115, die in Figur 13 mit der Bezugsziffer 115 angedeutet ist, erstreckt.

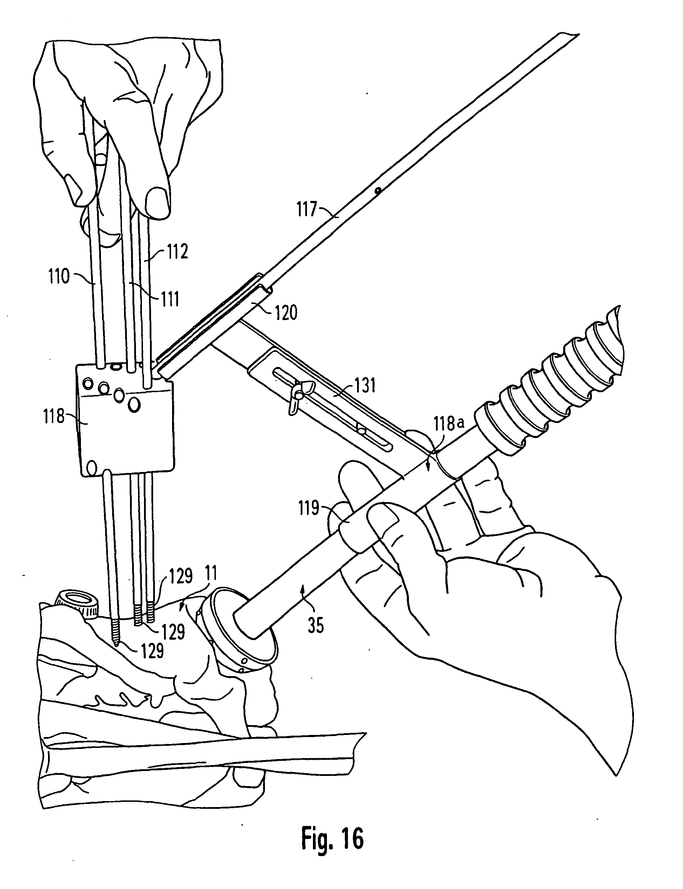

Am Führungsstab 117 ist ein Führungselement 118 a in Form einer Hülsenhälfte ansteckbar. Die Hülsenhälfte 119 dient zur Führung einer in Figur 16 nicht näher dargestellten Fräser-Antriebsachse 25 oder einer sich um diese herum erstreckende Hülse 26. Sie dient des weiteren zur Führung bzw. Ausrichtung eines Pfannen-Einschlaginstruments 35, wobei durch das Führungselement 118 a in Form der Hülsenhälfte 119 sichergestellt ist, daß die Ausrichtung der Fräser-Antriebsachse und des Pfanneneinschlaginstruments derjenigen der Manipulierpfanne 20 entspricht. In diesem Fall fallen die Manipulierpfannen-Mittenachse 115 mit der Längsachse der Fräser-Antriebsachse sowie der Längsachse des Pfannen-Einschlaginstruments zusammen.

Die als Führungselement dienende Hülsenhälfte 119 ist über einen flachen Bügel 131 mit dem Führungsstab 117 verbunden. Der Verbindungsbügel 131 ist bei der dargestellten Ausführungsform längenverstellbar. An dem dem Führungsstab 117 zugeordneten Ende des Verbindungsbügels 131 ist ebenfalls eine Hülsenhälfte 120 als Aufsteck- und Gleitschuh angeordnet. Damit läßt sich das Führungselement 118 a in einfacher Weise am Führungsstab 117 plazieren und längs desselben verschieben.

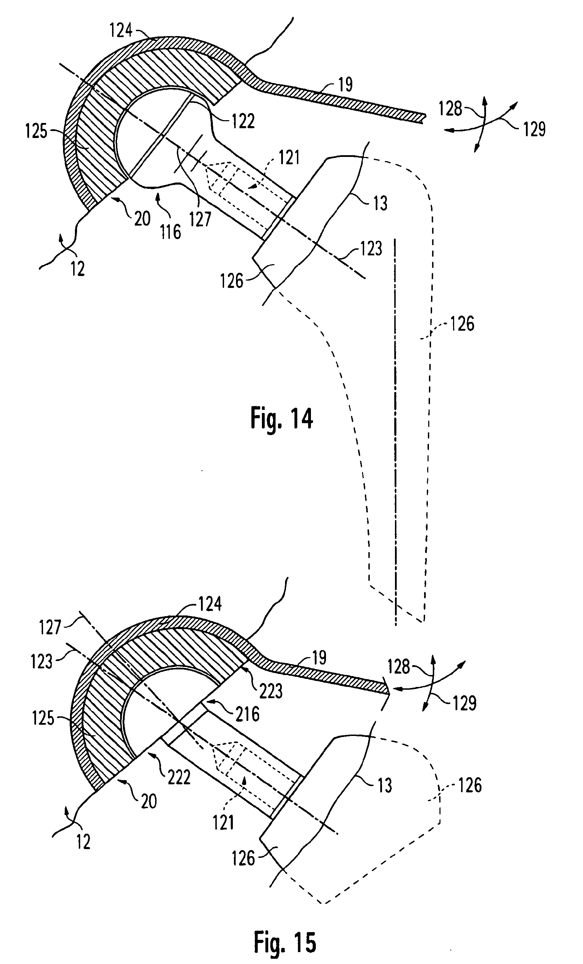

In den Figuren 14 und 15 sind noch zwei alternative Ausführungsbeispiele für einen Manipulier-Gelenkkopf 116 einerseits und 216 andererseits dargestellt. Beide Gelenkköpfe sind auf den Hals 121 einer Manipulierraspel 126 aufgesteckt. Die Manipulierraspel 126 ist innerhalb des proximalen Endes eines nicht näher dargestellten Femurs 13 plaziert. Die Längsachse des zapfenartigen Halses 121 entspricht der Schenkelhalsachse und ist mit der Bezugsziffer 123 gekennzeichnet. Die Manipulierpfanne 20 umfaßt eine metallene Außenschale 124, an der der Haltebügel 19 angeformt ist, sowie eine innere Kunststoffschale bzw. ein Inlay 125. Insofern entspricht die Manipulierpfanne 20 hinsichtlich ihres Aufbaus einer endgültig implantierbaren Hüftgelenkpfanne.

Bei dem in Figur 14 dargestellten Ausführungsbeispiel dienen als Mittel zur Ausrichtung der Manipulierpfanne 20 im Acetabulum 12 optische Erkennungsmittel 122. Konkret ist das optische Erkennungsmittel 122 als eine sich über den Umfang des sphährischen Teils des Manipulier-Gelenkkopfes 116 erstreckende Kerbe oder Nut ausgebildet. Diese erstreckt sich im vorliegenden Fall senkrecht zur Gelenkkopf-Mittenachse 127, die bei dem Ausführungsbeispiel gemäß Figur 14 mit der Schenkelhalsachse 123 zusammenfällt.

Die sich über den Umfang des sphärischen Teils des Manipulier-Gelenkkopfes 116 erstreckende Kerbe 122 liegt innerhalb der komplementären Lagerfläche der Manipulierpfanne 20, wenn die Gelenkkopf-Mittenachse 127 des Manipulier-Gelenkkopfes 116 mit der Manipulierpfannen-Mittenachse zusammenfällt. Erst durch relative Verschwenkung des Manipulier-Gelenkkopfes innerhalb der Manipulierpfanne 20 wird die Umfangskerbe 122 sichtbar. Dies ist ein Erkennungszeichen dafür, daß die Manipulierpfanne 20 korrekt ausgerichtet ist. Vorzugsweise weist der Manipulier-Gelenkkopf bzw. dessen sphärischer Teil und die Manipulierpfanne miteinander korrespondierende, sich jeweils in Umfangsrichtung erstreckende Markierungen auf, um die Ausrichtung der Manipulierpfanne auch um die Schenkelhalsachse 123 herum korrekt vornehmen zu können. Die möglichen Bewegungen der Manipulierpfanne 20, bei denen es sich um universalgelenkige Bewegungen handelt, sind in Figur 14 mit den Doppelpfeilen 128, 129 gekennzeichnet.

Ein drittes Ausführungsbeispiel für Mittel zur Ausrichtung der Manipulierpfanne 20 im Acetabulum 12 ist in Figur 15 schematisch dargestellt. Dort erfolgt die Ausrichtung der Manipulierpfanne 20 durch Zusammenspiel einer sich in einer Ebene senkrecht zur Gelenkkopf-Mittenachse 127 erstreckenden Umfangsschulter 222 mit einer sich schräg zur Gelenkkopf-Mittenachse 127 erstreckenden Aufnahme für den Hals 121 der Manipulierraspel 126, dessen - wie bereits erwähnt - Längsachse sich parallel zur Schenkelhalsachse 123 erstreckt.

Die korrekte Ausrichtung der Manipulierpfanne 20 ist dann erhalten, wenn die erwähnte Umfangsschulter 222 bündig ist mit der Umfangsringfläche 223 der Manipulierpfanne 20 bzw. des Inlays 125 derselben.

Die beiden letztgenannten Ausführungsbeispiele weisen also optische Erkennungsmittel für die Ausrichtung der Manipulierpfanne auf. Sie sind einfach und funktionssicher handhabbar.

Natürlich muß beim Ausführungsbeispiel gemäß Figur 15 auch darauf geachtet werden, daß der Manipulier-Gelenkkopf relativ zur Manipulierraspel 126 in vorbestimmter Weise ausgerichtet ist, d.h. die Gelenkkopf-Mittenachse 127 in eine vorgegebene Richtung weist. Nur dann ist sichergestellt, daß die eingangs gestellte Aufgabe beim endgültigen Implantat gelöst wird.

### Bezugszeichen

| | |
|---|---|
| 11 | Beckenknochen |
| 12 | Acetabulum |
| 13 | Femur |
| 14 | proximales Ende einer Manipulierraspel |
| 15 | Prothesenhals |
| 16 | Manipulier-Gelenkkopf |
| 17 | Führungsstab |
| 18 | Führungsblock |
| 19 | Haltebügel |
| 20 | Manipulierpfanne |
| 21 | sphärischer Teil des Manipulier-Gelenkkopfes |
| 22 | Schulter |
| 23 | Öffnungsrand |
| 24 | Ausrichtlehre |
| 25 | Fräser-Antriebsachse bzw. -welle |
| 26 | Drehhülse |
| 27 | Pfanneneinschlaginstrument |
| 28 | Fräserkopf |
| 29 | Gewinde |
| 30 | Führungshülse bzw. Führungselement |
| 31 | Bügel |
| 32 | Richtplatte |
| 33 | Markierung |
| 34 | Hüftpfanne |
| 36 | Führungsbohrung |
| 37 | Plattenschenkel |
| 38 | Plattenschenkel |
| 39 | Doppelpfeil |
| 40 | Einkerbung |
| 41 | Feststellschraube |
| 42 | Führungsbohrung |
| 110 | Fixierstab |
| 111 | Fixierstab |
| 112 | Fixierstab |
| 113 | Lochreihe |
| 114 | Lochreihe |
| 115 | Manipulierpfannen-Mittenachse |
| 116 | Manipulier-Gelenkkopf |
| 117 | Führungsstab |
| 118 | Haltevorrichtung |
| 118 a | Führungselement |
| 119 | Hülsenhälfte |
| 120 | Hülsenhälfte |
| 121 | Hals einer Manipulierraspel |
| 122 | Umfangskerbe |
| 123 | Halsachse/Schenkelhalsachse |
| 124 | Außenschale |
| 125 | Inlay |
| 126 | Manipulierraspel |
| 127 | Gelenkkopf-Mittenachse |
| 128 | Doppelpfeil |
| 129 | Doppelpfeil |
| 131 | Bügel |
| 216 | Manipulier-Gelenkkopf |
| 222 | Umfangsschulter |
| 223 | Umfangsringfläche |

## Patentansprüche

1. Hilfsmittel zur Implantation einer Hüftgelenkendoprothese, mit einer Manipulierpfanne (20), einem Manipulier-Gelenkkopf (16; 116, 216) und mit einer Einrichtung (17) zum Festhalten der ausgerichteten Lage der Manipulierpfanne (20),
**dadurch gekennzeichnet, daß**
der Manipulier-Gelenkkopf (16; 116, 216) mit Mitteln (22; 122; 222; 223) zur Ausrichtung der Manipulierpfanne (20) im Acetabulum (12) versehen ist, und daß die Einrichtung (17) zum Festhalten derart ausgebildet ist, daß mittels ihrer ein Knochenfräser (28) und/oder ein Einschlaginstrument (27) für die Plazierung der Hüftpfanne (34) ausrichtbar sind.

2. Hilfsmittel nach Anspruch 1,
**dadurch gekennzeichnet, daß**
der Manipulier-Gelenkkopf (16) eine über den sphärischen Teil (21) radial nach außen vorstehende Schulter (22) aufweist, die mit dem Öffnungsrand (23) der Manipulierpfanne (20) zur Ausrichtung derselben im Acetabulum (12) korrespondiert.

3. Hilfsmittel nach Anspruch 2,
**dadurch gekennzeichnet, daß**
die Schulter (22) durch etwa gleichmäßig über den Umfang verteilt angeordnete Schulterabschnitte definiert ist.

4. Hilfsmittel nach einem der Ansprüche 1 bis 3,
**dadurch gekennzeichnet, daß**
als Einrichtung zum Festhalten der ausgerichteten Lage der Manipulierpfanne (20) ein im Knochen (11) fixierbarer Führungsstab (17) dient, der mit einer an der Manipulierpfanne (20) angeordneten Führungseinrichtung (18) korrespondiert.

5. Hilfsmittel nach Anspruch 4,
**dadurch gekennzeichnet, daß** der Führungsstab (17) an dem im Knochen (11) verankerbaren Endabschnitt ein Schraubgewinde (29) aufweist, so daß er in den Knochen (11) einschraubbar ist.

6. Hilfsmittel nach Anspruch 4 oder 5,
**dadurch gekennzeichnet, daß**
die Führungseinrichtung (18) an der Manipulierpfanne (20) ein über einen Arm (19) mit der Manipulierpfanne (20) verbundenes Bauteil (Führungsblock 18 oder Führungshülse) mit einer Bohrung (36) zur Aufnahme und Führung des Führungsstabes (17) umfaßt.

7. Hilfsmittel nach einem der Ansprüche 4 bis 6,
**gekennzeichnet durch**
eine am Führungsstab (17) befestigbare, insbesondere auf diesen aufschiebbare Lehre (24) zur Ausrichtung eines Fräserkopfes (28) bzw. dessen Antriebsachse (25) so, daß die Ausrichtung des Fräserkopfes (28) derjenigen der Manipulierpfanne (20) entspricht.

8. Hilfsmittel nach Anspruch 7,
**dadurch gekennzeichnet, daß**
die Ausrichtlehre (24) einen auf den Führungsstab (17) aufschiebbaren Arm, insbesondere Bügel (31) umfaßt, an dessen freiem Ende eine Richtplatte (32), insbesondere eine mit Markierungen (0°; ± 5°) versehene Richtplatte (32) zur Ausrichtung der Fräser-Antriebsachse (25) angeordnet ist, wobei zur Ausrichtung der Fräser-Antriebsachse (25) diese in volle bzw. spaltfreie Anlage an die Richtplatte (32) und parallel zu dieser, insbesondere parallel zu dieser in eine Lage entsprechend einer vorgegebenen Markierung (0°; ± 5°; 40), insbesondere vorgegebene Nullposition, verschwenkt wird.

9. Hilfsmittel nach Anspruch 8,
**dadurch gekennzeichnet, daß** das Hilfsmittel einen Fräskopf (28) mit einer Fräser-Antriebsachse (25) aufweist, auf die eine Hülse (26) aufsteckbar ist, innerhalb der die Antriebsachse (25) drehbar gelagert und an der die Richtplatte (32) in volle bzw. spaltfreie Anlage bring- und während des Fräsens haltbar ist.

10. Hilfsmittel nach einem der Ansprüche 1 bis 9,
**gekennzeichnet durch**
ein Pfanneneinschlaginstrument (27), welches an der Richtplatte (32) der Ausrichtlehre (24) in gleicher Weise wie der Fräserkopf (28) bzw. dessen Antriebsachse (25) ausrichtbar ist.

11. Hilfsmittel nach einem der Ansprüche 1 bis 10,
**dadurch gekennzeichnet, daß**
die Manipulierpfanne (20) mit einer Führungseinrichtung (18) für zwei parallel zueinander im Knochen (11) fixierbare Führungsstäbe (17) versehen ist (Figur 12).

12. Hilfsmittel nach Anspruch 11,
**dadurch gekennzeichnet, daß**
die Lehre (24) zur Ausrichtung des Fräserkopfes bzw. dessen Antriebsachse (25) zwei Durchgangsbohrungen (42) zum Aufschieben auf die im Knochen fixierten Führungsstäbe (17) aufweist.

13. Hilfsmittel nach einem der Ansprüche 8 bis 12,
**dadurch gekennzeichnet, daß**
die Richtplatte (32) der Ausrichtlehre (24) U-förmig gebogen ist, wobei der Raum zwischen den beiden Plattenschenkeln (37, 38) zur Aufnahme der Fräser-Antriebsachse (25) und/oder des Pfanneneinschlaginstruments (27) dient.

14. Hilfsmittel nach Anspruch 13,
**dadurch gekennzeichnet, daß**
der dem Operateur zugewandte, insbesondere obere Plattenschenkel (37) stirnseitig Einkerbungen (40) als Markierungen für die Ausrichtung der Fräser-Antriebsachse (25) und/oder des Pfanneneinschlaginstruments (27) parallel zur Richtplatte (32) bzw. deren Plattenschenkel (37, 38) aufweist.

15. Hilfsmittel nach einem der Ansprüche 1 bis 14,
**dadurch gekennzeichnet, daß**
der Manipulier-Gelenkkopf (16) am Hals (15) einer Manipulierraspel (14) befestigbar, insbesondere auf diesen aufsteckbar ist.

16. Hilfsmittel nach Anspruch 1,
**dadurch gekennzeichnet, daß**
als Mittel zur Ausrichtung der Manipulierpfanne (20) im Acetabulum (12) optische Erkennungsmittel (122; 222) dienen.

17. Hilfsmittel nach Anspruch 16,
**dadurch gekennzeichnet, daß**
der sphärische Teil des Manipulier-Gelenkkopfes (116) eine sich zumindest teilweise über den Umfang erstreckende Markierung, insbesondere Kerbe (122), Nut oder dgl. aufweist, die sich innerhalb einer Ebene erstreckt, die entweder senkrecht oder in einem vorbestimmten Winkel zur Gelenkkopf-Mittenachse (127) liegt.

18. Hilfsmittel nach Anspruch 1,
**dadurch gekennzeichnet, daß**
als Mittel zur Ausrichtung der Manipulierpfanne (20) im Acetabulum (12) eine sich in einer Ebene senkrecht zur Gelenkkopf-Mittenachse (127) erstreckende Umfangsschulter (222) in Verbindung mit einer sich schräg zur Gelenkkopf-Mittenachse (127) erstreckenden Aufnahme für den Hals (121) einer Manipulierraspel (126) dient, dessen Längsachse sich parallel zur Schenkelhalsachse (123) erstreckt.

19. Hilfsmittel nach einem der Ansprüche 1 bis 3 und/oder 16 bis 18,
**dadurch gekennzeichnet, daß**
als Einrichtung zum Festhalten der ausgerichteten Lage der Manipulierpfanne (20) mindestens ein, insbesondere drei Fixierstäbe (110, 111, 112) dient bzw. dienen, der bzw. die sich durch eine Haltevorrichtung (118) für die Manipulierpfanne (20) hindurch erstreckt bzw. hindurch erstrecken.

20. Hilfsmittel nach Anspruch 19,
**dadurch gekennzeichnet, daß**
der bzw. die Fixierstäbe (110, 111, 112) an den im Knochen verankerbaren Endabschnitten Schraubgewinde (29) aufweisen, so daß sie in den Knochen (11) einschraubbar sind.

21. Hilfsmittel nach Anspruch 19 oder 20,
**dadurch gekennzeichnet, daß**
an der Haltevorrichtung (118) ein Führungsstab (117) anschließbar ist so, daß dieser sich etwa parallel zur Manipulierpfannen-Mittenachse (115) erstreckt.

22. Hilfsmittel nach Anspruch 21,
**gekennzeichnet durch**
ein am Führungsstab (117) an- oder aufsteckbares Führungselement (118) für eine Fräser-Antriebsachse (25) oder eine sich um diese herum erstreckende Hülse (26) sowie für ein Pfannen-Einschlaginstrument (35), wobei **durch** das Führungselement (118) sichergestellt ist, daß die Ausrichtung der Fräser-Antriebsachse (25) und des Pfannen-Einschlaginstruments (35) derjenigen der Manipulierpfanne (20) entspricht.

23. Hilfsmittel nach Anspruch 22,
**dadurch gekennzeichnet, daß**
das Führungselement (118) eine an einem Bügel (131) oder dergleichen Verbindungselement angeordnete Hülse oder Hülsenhälfte (119) ist.

24. Hilfsmittel nach Anspruch 23,
**dadurch gekennzeichnet, daß**
der Bügel (131) längenverstellbar ist.

## Claims

1. Aid for implantation of a hip joint endoprosthesis, having a manipulation cup (20), a manipulation joint head (16; 116, 216) and having a device (17) for retaining the aligned position of the manipulation cup (20),
**characterized in that**
the manipulation joint head (16; 116, 216) is provided with means (22; 122; 222; 223) for aligning the manipulation cup (20) in the acetabulum (12), and the retaining device (17) is constructed in such a way that by means of that device a bone milling cutter (28) and/or an impaction instrument (27) is alignable for placement of the acetabular cup (34).

2. Aid according to claim 1,
**characterized in that**
the manipulation joint head (16) has a shoulder (22) which projects radially outwards beyond the spherical portion (21), which shoulder has correspondence with the edge (23) of the opening of the manipulation cup (20) for alignment thereof in the acetabulum (12).

3. Aid according to claim 2,
**characterized in that**
the shoulder (22) is defined by shoulder portions distributed approximately uniformly around the periphery.

4. Aid according to any one of claims 1 to 3,
**characterized in that**
as the device for retaining the aligned position of the manipulation cup (20) there is used a guide rod (17), fixable in the bone (11), which has correspondence with a guide device (18) arranged on the manipulation cup (20).

5. Aid according to claim 4,
**characterized in that**
the guide rod (17) has a screw thread (29) on its end portion anchorable in the bone (11) so that it can be screwed into the bone (11).

6. Aid according to claim 4 or 5,
**characterized in that**
the guide device (18) on the manipulation cup (20) comprises a component (guide block 18 or guide sleeve) connected by means of an arm (19) to the manipulation cup (20) and having a bore (36) for receiving and guiding the guide rod (17).

7. Aid according to any one of claims 4 to 6,
**characterized by**
a jig (24), attachable to, especially slidable onto, the guide rod (17), for alignment of a milling cutter head (28) or the drive shaft (25) thereof in such a way that the alignment of the milling cutter head (28) corresponds to that of the manipulation cup (20).

8. Aid according to claim 7,
**characterized in that**
the alignment jig (24) comprises an arm, especially a holder bar (31), slidable onto the guide rod (17), there being arranged on the free end of the arm an orienting plate (32), especially an orienting plate (32) provided with markings (0°; ± 5°) for alignment of the milling cutter drive shaft (25), wherein for alignment of the milling cutter drive shaft (25) the latter is pivoted into full, that is to say gap-free, contact with the orienting plate (32) and parallel thereto, especially parallel thereto into a position corresponding to a preset marking (0°; ± 5°; 40), especially a preset zero position.

9. Aid according to claim 8,
**characterized in that**
the aid has a milling head (28) having a milling cutter drive shaft (25) on which a sleeve (26) is mountable, inside which sleeve the drive shaft (25) is rotatably mounted and with which the orienting plate (32) can be brought into full, that is to say gap-free, contact and against which it can be held during the milling.

10. Aid according to any one of claims 1 to 9,
**characterized by**
a cup impaction instrument (27) which is alignable on the orienting plate (32) of the alignment jig (24) in the same way as the milling cutter head (28) or the drive shaft (25) thereof.

11. Aid according to any one of claims 1 to 10,
**characterized in that**
the manipulation cup (20) is provided with a guide device (18) for two guide rods (17) which are fixable in the bone (11) in parallel with one another (Figure 12).

12. Aid according to claim 11,
**characterized in that**
the jig (24) has, for alignment of the milling cutter head or the drive shaft (25) thereof, two through-bores (42) for sliding onto the guide rods (17) fixed in the bone.

13. Aid according to any one of claims 8 to 12,
**characterized in that**
the orienting plate (32) of the alignment jig (24) is curved into a U-shape, the space between the two limbs (37, 38) of the plate serving to receive the milling cutter drive shaft (25) and/or the cup impaction instrument (27).

14. Aid according to claim 13,
**characterized in that**
the plate limb that faces the surgeon, especially the upper plate limb (37), has notchings (40) on its end face as markings for the alignment of the milling cutter drive shaft (25) and/or of the cup impaction instrument (27) parallel to the orienting plate (32) or the plate limbs (37, 38) thereof.

15. Aid according to any one of claims 1 to 14,
**characterized in that**
the manipulation joint head (16) is attachable to, especially mountable on, the neck (15) of a manipulation rasp (14).

16. Aid according to claim 1,
**characterized in that**
the means used for aligning the manipulation cup (20) in the acetabulum (12) are optical recognition means (122; 222).

17. Aid according to claim 16,
**characterized in that**
the spherical portion of the manipulation joint head (116) has a marking, especially a notch (122), groove or the like, extending around at least part of the periphery, which marking extends in a plane lying either perpendicular to or at a predetermined angle with respect to the centre axis (127) of the joint head.

18. Aid according to claim 1,
**characterized in that**
as means for alignment of the manipulation cup (20) in the acetabulum (12) there is used a peripheral shoulder (222), which extends in a plane perpendicular to the joint head centre axis (127), in conjunction with a seating, which extends obliquely with respect to the joint head centre axis (127), for the neck (121) of a manipulation rasp (126), the longitudinal axis of which runs parallel to the shank neck axis (123).

19. Aid according to any one of claims 1 to 3 and/or 16 to 18,
**characterized in that**
as device for retaining the aligned position of the manipulation cup (20) there is used at least one, especially three, fixing rod(s) (110, 111, 112) which extend(s) through a holding device (118) for the manipulation cup (20).

20. Aid according to claim 19,
**characterized in that**
the fixing rod(s) (110, 111, 112) have screw threads (29) on their end portions anchorable in the bone, so that they can be screwed into the bone (11).

21. Aid according to claim 19 or 20,
**characterized in that**
a guide rod (117) is connectible to the holding device (118) in such a way that it extends approximately parallel to the centre axis (115) of the manipulation cup.

22. Aid according to claim 21,
**characterized by**
a guide element (118), attachable to or mountable on the guide rod (117), for a milling cutter drive shaft (25) or a sleeve (26) extending around the latter and for a cup impaction instrument (35), it being ensured by means of the guide element (118) that the alignment of the milling cutter drive shaft (25) and of the cup impaction instrument (35) corresponds to that of the manipulation cup (20).

23. Aid according to claim 22,
**characterized in that**
the guide element (118) is a sleeve or half-sleeve (119) arranged on a holder bar (131) or like connecting element.

24. Aid according to claim 23,
**characterized in that**
the holder bar (131) is adjustable in length.

## Revendications

1. Accessoire pour l'implantation d'une prothèse de la hanche, comportant une cavité de manipulation (20), une tête articulée de manipulation (16 ; 116, 216) et comportant un dispositif (17) destiné à immobiliser la position ajustée de la cavité de manipulation (20), **caractérisé en ce que** la tête articulée de manipulation (16 ; 116, 216) est munie de moyens (22 ; 122 ; 222 ; 223) pour ajuster la cavité de manipulation (20) dans le cotyle (12), et **en ce que** le dispositif (17) destiné à immobiliser est réalisé de telle sorte qu'au moyen de celui-ci, une fraise à os (28) et/ou un impacteur (27) peuvent être ajustés pour la mise en place de la pièce acétabulaire (34).

2. Accessoire selon la revendication 1, **caractérisé en ce que** la tête articulée de manipulation (16) comporte un épaulement (22), qui s'avance radialement vers l'extérieur au-delà de la partie sphérique (21) et qui correspond au bord d'ouverture (23) de la cavité de manipulation (20) pour l'ajustement de celle-ci dans le cotyle (12).

3. Accessoire selon la revendication 2, **caractérisé en ce que** l'épaulement (22) est défini par des tronçons d'épaulement disposés en étant répartis sensiblement uniformément sur le pourtour.

4. Accessoire selon l'une quelconque des revendications 1 à 3, **caractérisé en ce que** pour former le dispositif destiné à immobiliser la position ajustée de la cavité de manipulation (20), on utilise une tige de guidage (17) pouvant être fixée dans l'os (11) et coopérant avec un dispositif de guidage (18) disposé dans la cavité de manipulation (20).

5. Accessoire selon la revendication 4, **caractérisé en ce que** la tige de guidage (17), au niveau de la zone d'extrémité apte à être ancrée dans l'os (11), comporte un filetage (29), de telle sorte qu'elle peut être vissée dans l'os (11).

6. Accessoire selon la revendication 4 ou 5, **caractérisé en ce que** le dispositif de guidage (18) sur la cavité de manipulation (20) comporte une pièce (bloc de guidage 18 ou manchon de guidage), qui est reliée à la cavité de manipulation (20) par l'intermédiaire d'un bras (19) et qui comporte une forure (36) destinée à recevoir et à guider la tige de guidage (17).

7. Accessoire selon l'une quelconque des revendications 4 à 6, **caractérisé par** un gabarit (24), qui peut être fixé sur la tige de guidage (17), en particulier peut être emmanché sur celle-ci, et qui est destiné à ajuster une tête de fraise (28) ou l'axe d'entraînement (25) de celle-ci, de telle sorte que l'ajustement de la tête de fraise (28) correspond à celui de la cavité de manipulation (20).

8. Accessoire selon la revendication 7, **caractérisé en ce que** le gabarit d'ajustement (24) comporte un bras, en particulier un étrier (31), qui peut être emmanché sur la tige de guidage (17) et sur l'extrémité libre duquel est disposée une plaque de référence (32), en particulier une plaque de référence (32) munie de repères (0° ; ± 5°), destinée à ajuster l'axe d'entraînement (25) de la fraise, sachant que pour l'ajustement de l'axe d'entraînement (25) de la fraise, on fait pivoter celui-ci pour venir en appui complet, c'est-à-dire sans interstice, contre la plaque de référence (32) et parallèlement à celle-ci, notamment parallèlement à celle-ci dans une position correspondant à un repère (0° ; ± 5° ; 40) prédéfini, en particulier dans la position zéro prédéfinie.

9. Accessoire selon la revendication 8, **caractérisé en ce que** ledit accessoire comporte une tête de fraise (28) avec un axe d'entraînement (25) de la fraise, sur laquelle peut être emmanchée un manchon (26), à l'intérieur duquel l'axe d'entraînement (25) est monté rotatif et sur lequel la plaque de référence (32) peut être amenée en appui complet, c'est-à-dire sans interstice, et peut être maintenue pendant le fraisage.

10. Accessoire selon l'une quelconque des revendications 1 à 9, **caractérisé par** un impacteur (27), qui peut être ajusté sur la plaque de référence (32) du gabarit d'ajustement (24) de la même manière que la tête de fraisage (28) ou l'axe d'entraînement (25) de celle-ci.

11. Accessoire selon l'une quelconque des revendications 1 à 10, **caractérisé en ce que** la cavité de manipulation (20) est munie d'un dispositif de guidage (18) pour deux tiges de guidage (17) aptes à être fixées parallèlement entre elles dans l'os (11) (figure 12).

12. Accessoire selon la revendication 11, **caractérisé en ce que** le gabarit (24), destiné à ajuster la tête de fraisage (28) ou l'axe d'entraînement (25) de celle-ci, comporte deux forures débouchantes (42) pour l'emmancher sur les tiges de guidage (17) fixées dans l'os.

13. Accessoire selon l'une quelconque des revendications 8 à 12, **caractérisé en ce que** la plaque de référence (32) du gabarit d'ajustement (24) est pliée en forme de U, l'espace entre les deux branches (37, 38) étant destiné à recevoir l'axe d'entraînement (25) de la fraise et/ou l'impacteur (27).

14. Accessoire selon la revendication 13, **caractérisé en ce que** la branche (37) orientée vers l'opérateur, en particulier la branche supérieure, comporte sur la face frontale des encoches (40) formant des marquages pour l'ajustement de l'axe d'entraînement (25) de la fraise et/ou de l'impacteur (27) parallèlement à la plaque de référence (32) ou aux branches (37, 38) de celle-ci.

15. Accessoire selon l'une quelconque des revendications 1 à 14, **caractérisé en ce que** la tête articulée de manipulation (16) peut être fixée sur le manche (15) d'une râpe de manipulation (14), en particulier peut être emmanchée sur celui-ci.

16. Accessoire selon la revendication 1, **caractérisé en ce que** pour les moyens destinés à ajuster la cavité de manipulation (20) dans le cotyle (12), on utilise des moyens de détection (122 ; 222) optiques.

17. Accessoire selon la revendication 16, **caractérisé en ce que** la partie sphérique de la tête articulée de manipulation (116) comporte un marquage, en particulier une encoche (122), rainure ou élément similaire, qui s'étend au moins en partie sur tout le pourtour et qui s'étend à l'intérieur d'un plan qui est situé perpendiculairement ou selon un angle prédéfini par rapport à l'axe médian (127) de la tête articulée.

18. Accessoire selon la revendication 1, **caractérisé en ce que** pour les moyens destinés à ajuster la cavité de manipulation (20) dans le cotyle (12), on utilise un épaulement périphérique (222), qui s'étend dans un plan perpendiculaire à l'axe médian (127) de la tête articulée, en association avec un logement, orienté en oblique par rapport à l'axe médian (127) de la tête articulée et destiné à recevoir le manche (121) d'une râpe de manipulation (126), dont l'axe longitudinal est parallèle à l'axe du manche (123).

19. Accessoire selon l'une quelconque des revendications 1 à 3 et/ou 16 à 18, **caractérisé en ce que** pour le dispositif destiné à immobiliser la position ajustée de la cavité de manipulation (20), on utilise au moins une, en particulier trois tiges de fixation (110, 111, 112), laquelle ou lesquelles s'étend(ent) à travers un dispositif de fixation (118) pour la cavité de manipulation (20).

20. Accessoire selon la revendication 19, **caractérisé en ce que** la ou les tiges de fixation (110, 111, 112), au niveau des zones d'extrémité pouvant être ancrées dans l'os, comportent un filetage (29), de telle sorte qu'elles peuvent être vissées dans l'os (11).

21. Accessoire selon la revendication 19 ou 20, **caractérisé en ce qu'**une tige de guidage (117) peut être attachée au dispositif de fixation (118), de telle sorte que ladite tige de guidage est sensiblement parallèle à l'axe médian (115) de la cavité de manipulation.

22. Accessoire selon la revendication 21, **caractérisé par** une tige de guidage (117) pouvant être fixée ou emmanchée sur l'élément de guidage (118) pour un axe d'entraînement (25) de la fraise ou un manchon (26) qui s'étend autour de celui-ci, ainsi que pour un impacteur (35), sachant que l'élément de guidage (118) permet de garantir que l'ajustement de l'axe d'entraînement (25) de la fraise et de l'impacteur (35) correspond à celui de la cavité de manipulation (20).

23. Accessoire selon la revendication 22, **caractérisé en ce que** l'élément de guidage (118) est un manchon ou un demi-manchon (119), disposé sur un étrier (131) ou un élément d'assemblage similaire.

24. Accessoire selon la revendication 23, **caractérisé en ce que** l'étrier (131) est réglable en longueur.
